# EUROPEAN PATENT SPECIFICATION

(11) **EP 0 506 235 B1**
(45) Date of publication and mention of the grant of the patent: **28.04.1999**
(21) Application number: 92301614.1
(22) Date of filing: 26.02.1992
(51) Int. Cl.: C07D 317/26, C07D 317/20, C07D 407/04

(54) **Glyceraldehyde-3-pentanide and process for producing same**
Glyceraldehyd-3-pentanid und Verfahren zu ihrer Herstellung
3-Pentanide de glycéraldéhyde et procédé pour sa préparation

(30) Priority: 28.02.1991 US 661964
(43) Date of publication of application: 30.09.1992
(73) Proprietor: ELI LILLY AND COMPANY, Indianapolis, Indiana 46285 (US)
(72) Inventor: Schmid, Christopher Randall, Indianapolis, Indiana 46220 (US)
(74) Representative: Hudson, Christopher Mark

(56) References cited:
- EP-A- 0 120 289
- EP-A- 0 290 744
- Agric.Biol.Chem.48(7),1841-1844,1984
- J.Chem.Soc.,Perkin Trans. I,1986(5),765-770

## Description

This invention relates to a process for producing glyceraldehyde-3-pentanide.

The (R)- and (S)-stereoisomers of glyceraldehyde acetonide are useful as starting materials in the synthesis of numerous drugs, agricultural chemicals and naturally occurring substances. For example, United States Patent No. 4,526,988 discloses that (R)-glyceraldehyde acetonide is a preferred substrate for preparing 2'-deoxy-2',2'-difluoronucleosides having antiviral activity. European Patent Application 184,365 teaches that the same difluoronucleosides also have oncolytic activity. Finally, United States Patent No. 4,567,282 discloses that (S)-glyceraldehyde acetonide can be used to synthesize the antiepileptic, antihypertensive agent (R)-(-)-γ-amino-β-hydroxybutyric acid, as well as various antibiotics.

Using glyceraldehyde acetonide to produce the above-mentioned compounds on a production scale has several disadvantages. Firstly, the known processes for producing the stereoisomers of glyceraldehyde acetonide generally provide such stereoisomers in low yield when employed on a production scale. Furthermore, glyceraldehyde acetonide possesses poor monomeric stability due to the compound's tendency to polymerize. Polymerization can be prevented by dissolving the acetonide in water. However, if the next reaction step in the synthesis of the desired drug, agricultural chemical or naturally occurring substance cannot employ aqueous reaction conditions, recovery of glyceraldehyde acetonide from such water solution is extremely difficult. Accordingly, polymerization precludes the possibility of producing and storing neat glyceraldehyde acetonide until needed, and the difficulty of recovering glyceraldehyde acetonide from aqueous solutions of same renders undersirable the possibility of producing and storing such compound as an aqueous solution in those instances wherein conversion of glyceraldehyde acetonide to the desired end product requires non-aqueous reaction conditions in the next step of product synthesis. Such polymerization and poor aqueous recoverability concerns limit production flexibility.

EP-A-0290744 discloses the preparation of R-glyceraldehyde-3-pentanide by the reaction of 3-pentanone with D-mannitol and the subsequent glycol cleavage of the so formed (di-)ketalised D-mannitol intermediate by periodic acid in methanol-water solution.

EP-A-0120289 discloses the preparation of S-glyceraldehyde-acetonide.

Agric. Biol. Chem., 48(7), 1841-1844, 1984 discloses the preparation of 2,3-O-cyclohexyliden-D-glyceraldehyde in an attempt to obtain a glyceraldehyde derivative with an enhanced stability.

J. Chem. Soc. Perkin Trans. I, 1986(5), 765-770 discloses the use of a potassium periodate/sodium hydroxide-system in the conversion of 1,2:5,6-di-O-isopropylidene-D-mannitol to the corresponding glyceraldehyde-acetonide.

The present invention provides a novel process for preparing glyceraldehyde-3-pentanide. Such compound can be used to synthesize the drugs, agricultural chemicals and naturally occurring substances described in United States Patent Nos. 4,526,988 and 4,567,282.

Accordingly, one object of the present invention is to provide a process for the preparation of a glyceraldehyde substrate on a production scale in high yield using commercially feasible technology.

Thus, the invention provides a process for preparing glyceraldehyde-3-pentanide which comprises reacting a compound of the formula 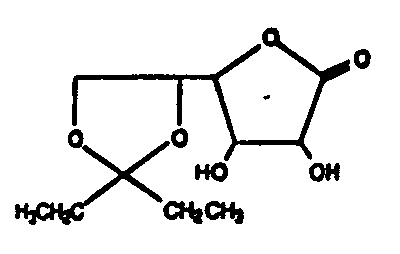 with a cleavage reagent selected from the group consisting of
a) potassium periodate and a base, and
b) dipotassium paraperiodate;
   wherein the pH of the reaction solution is maintained at between about 5.0 to about 9.0.

Glyceraldehyde-3-pentanide may be prepared using a two-step process from compounds which are well-known to those skilled in the art, and, in several instances, commercially available. This two-step process is set forth in Reaction Scheme I, below.

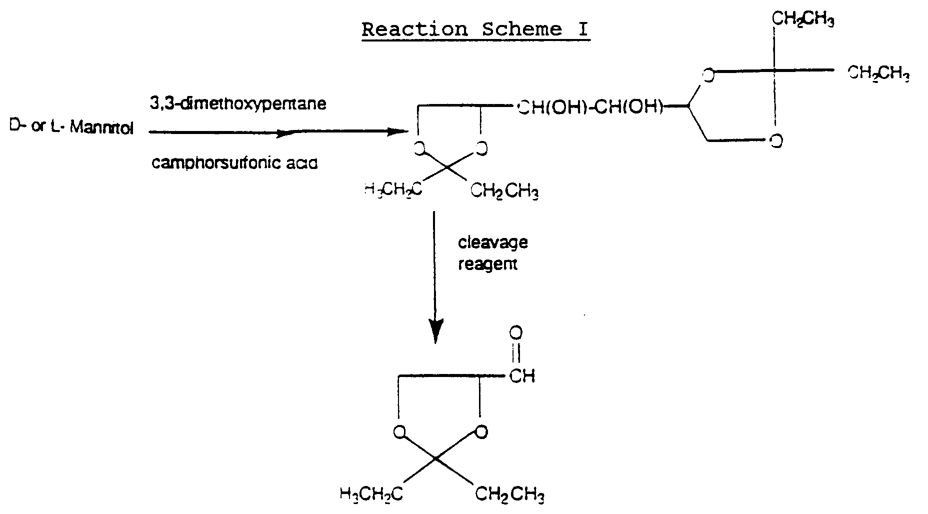

In the first step of the process of Reaction Scheme I, D- or L-mannitol is ketalized using 3,3-dimethoxypentane, with a catalytic amount of camphorsulfonic acid, to give a 1,2:5,6-diketalized product. Solvent choice for the reaction is not critical so long as the reactants are sufficiently solubilized to effect the desired reaction and the solvent is inert to the reaction conditions employed. A preferred solvent is dimethylformamide.

The concentration of mannitol and dimethoxypentane in the inert solvent is not critical. In general, it is desirable to use as concentrated a solution as possible in order to minimize any product loss which might occur during product isolation. However, enough solvent should be employed to ensure the reactants remain substantially in solution during the reaction.

The relative amounts of mannitol and dimethoxypentane employed are also not critical. In general, at least two moles of the dimethoxypentane reagent, or a slight excess thereof, relative to the mannitol reagent are employed in order to ensure that complete reaction of the mannitol reagent is obtained. Excessive amounts of dimethoxypentane should generally be avoided since use of such amounts will result in the formation of unwanted 1,2:3,4:5,6 triketalized product. Ideally, about 2.0 to 2.5 molar equivalents of 3,3-dimethoxypentane are employed relative to the mannitol reagent, with 2.1 molar equivalents being most preferred.

The ketalization reaction is generally conducted at a temperature of from about room temperature to the reflux temperature of the reaction mixture. The reaction is generally substantially complete after about one hour to about 72 hours when conducted at a temperature in the range described above. A reaction temperature of about 40°C is most preferred since formation of unwanted side products is best controlled at such temperature.

The diketalized product produced by the first step of the process of Reaction Scheme I can be isolated by standard methods, if desired. If isolated, the compound can be purified by recrystallization from any one of a variety of solvents. However, since purification can be both solvent and labor intensive, the crude isolated compound may be employed in the second step of the process of Reaction Scheme I directly.

Step one of Reaction Scheme I illustrates the use of D- or L-mannitol to prepare a 1,2:5,6-diketalized product. An alternative substrate which may be employed in place of the mannitol reagent is D- or L-gulono-1,4-lactone. Such alternate substrate, when reacted according to the reaction conditions described above, will provide a monoketalized product having the formula 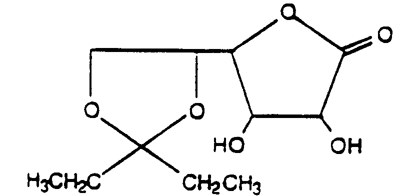 This monoketalized product may also be employed as a reactant in step two of the process of Reaction Scheme I.

When preparing R-glyceraldehyde-3-pentanide, D-mannitol is the preferred starting material. However, when preparing S-glyceraldehyde-3-pentanide, L-gulono-1,4-lactone is the substrate of choice.

The second step of the process detailed in Reaction Scheme I entails using a cleavage reagent to cleave the mono- or diketalized compound prepared in step one. Cleavage reagents suitable for use in this step include lead tetraacetate and the periodates such as lithium periodate, sodium periodate or potassium periodate. Potassium periodate is a preferred cleavage reagent since it is particularly useful for providing a process for preparing glyceraldehyde-3-pentanide which is readily adaptable to a production scale. Potassium periodate provides such adaptability because it is unique among the alkali metal periodates in terms of its relatively low aqueous solubility. This low solubility results in a solution pH of between pH 5 to pH 6, thereby affording a solution having a particularly desired pH range. Furthermore, potassium periodate's low aqueous solubility enables relatively easy reaction exotherm control.

The amount of cleavage reagent required to effect cleavage of the mono- or diketalized compound is not crucial. In general, at least an equimolar amount of the cleavage reagent, or a slight excess thereof, relative to the diketalized material is employed in order to ensure that complete reaction of the ketalized substrate is obtained. If cleavage of more than one bond is required in order to provide glyceraldehyde-3-pentanide (as is the case when the monoketalized substrate is employed) at least two moles of the cleavage reagent, or a slight excess thereof, relative to the monoketalized material should be employed.

The cleavage reaction of Reaction Scheme I is generally run in an inert solvent or combination of inert solvents. Solvent choice for the reaction is not critical so long as the reactants are sufficiently solubilized to effect the desired reaction and the solvent is inert to the reaction conditions employed. If lead tetraacetate is used as the cleavage reagent the inert solvent should generally be an organic solvent such as an ether, ketone or ester, for example. If a periodate is employed as the cleavage reagent the cleavage reaction should generally be run in water.

In the case of the periodate cleavage reagents the use of an organic co-solvent to increase reactant solubility may be desirable. Suitable co-solvents include all organic solvents which increase the reactants' solubility in water, are inert to the reaction conditions employed and are either totally or partially miscible in water. Typical examples of suitable co-solvents include ethers such as tetrahydrofuran, diethyl ether, ketones such as acetone, methyl ethyl ketone and esters such as methyl acetate. Tetrahydrofuran and acetone are preferred co-solvents.

The ratio of water to co-solvent is not critical. In general, the co-solvent should be employed in an amount sufficient to provide the desired degree of reactant solubility in water. Excessive amounts of co-solvent should be avoided. Typical water to co-solvent ratios which may be employed range from about 1:1 (v:v) water/co-solvent to about 10:1 (v:v) water/co-solvent. These ranges are provided for exemplary purposes, however, and are not meant to limit the scope of the present invention in any respect.

The concentration of di- or monoketalized compound and cleavage reactant in the inert solvent is not critical. In general, it is desirable to use as concentrated a solution as possible in order to minimize any product loss which might occur during product isolation. However, since the cleavage reaction is exothermic, enough solvent should be employed so that the reaction exotherm remains controllable.

The cleavage reaction is generally conducted at a temperature of from about 0°C to about 50°C. The reaction is generally substantially complete after about 15 minutes to about 8 hours when conducted at a temperature in the range described above. The progress of the reaction can be followed, if desired, by standard analytical techniques such as nuclear magnetic resonance (NMR).

To minimize ketal hydrolysis of the glyceraldehyde-3-pentanide product of step two of Reaction Scheme I, the reaction solution pH should be maintained at a pH of from about 5.0 to about 9.0. When certain of the periodates, for example sodium periodate, are employed as cleavage reagent, a reaction solution pH of less than 4.0 is obtained when such reagents are dissolved in water. To minimize ketal hydrolysis when cleavage reagents such as sodium periodate are utilized, one normally would think of adding a base to the reaction mixture in an amount sufficient to increase the reaction solution pH to between about 5.0 to about 9.0. However, addition of base to a cleavage reaction solution containing sodium periodate causes disodium paraperiodate to precipitate, thus decreasing reaction yield and, if enough base is added, terminating the cleavage reaction altogether.

Potassium periodate, because of its relatively low solubility in water, provides a reaction solution pH of about pH 5-6 when employed as cleavage reagent in the present process. As such, a cleavage reaction solution containing potassium periodate naturally has a reaction solution pH sufficient to minimize ketal hydrolysis (i.e., a reaction solution pH of from about 5.0 to about 9.0). Accordingly, potassium periodate is the preferred cleavage reagent.

However, even if potassium periodate is employed as cleavage reagent some ketal hydrolysis of the product of the cleavage reaction may occur. Formic acid can be produced in the cleavage reaction by cleavage of deketalized material. As formic acid is produced the reaction solution pH decreases. Eventually, and even if potassium periodate is employed as the cleavage reagent, the reaction solution pH will drop below pH 5.0, causing ketal hydrolysis.

To prevent formic acid induced ketal hydrolysis in cleavage reaction solutions containing potassium periodate, a base should be added in an amount sufficient to maintain the reaction solution's pH between about 5.0 to about 9.0 at all times during the cleavage reaction. Base addition may be accomplished by adding the base to the reaction mixture at the start of the reaction or by adding base throughout the reaction, as needed, to maintain the reaction solution pH at the desired pH value. Typical bases which may be used to minimize ketal hydrolysis include monobasic potassium phosphate, dibasic potassium phosphate, tri-basic potassium phosphate, the carbonates such as potassium carbonate, potassium bicarbonate, amines such as triethylamine and potassium hydroxide. Potassium hydroxide and potassium bicarbonate are preferred bases for use in the present process.

While addition of base to a cleavage reaction solution containing sodium periodate causes disodium paraperiodate to precipitate, addition of base to a cleavage reaction solution containing potassium periodate forms dipotassium paraperiodate. Dipotassium paraperiodate has greater aqueous solubility than potassium periodate. Thus, addition of base to a cleavage reaction solution containing potassium periodate actually increases the amount of periodate oxidant in solution, thereby increasing the rate of cleavage reaction relative to that which would be obtained using a base-free solution. Accordingly, base addition to cleavage reaction solutions containing potassium periodate is preferred for the process of the present invention since such base addition not only prevents formic acid induced ketal hydrolysis, but also increases cleavage reaction rate.

The glyceraldehyde-3-pentanide product of step two of Reaction Scheme I can be isolated using standard isolation techniques. Purification of such compound may also be accomplished using standard techniques, if desired.

R-Glyceraldehyde-3-pentanide may be produced from D-gulono-1,4-lactone using the process set forth in Reaction Scheme I. The use of such substrates produces intermediates of the formula 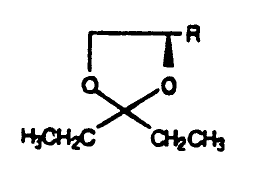 wherein R is

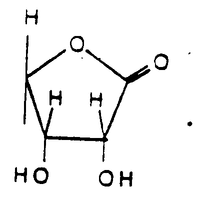

Likewise, S-glyceraldehyde-3-pentanide may be produced from L-gulono-1,4-lactone using the process of Reaction Scheme I. The use of such substrates produces intermediates of the formula 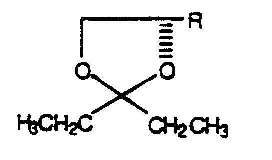 wherein R is 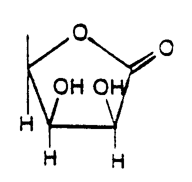 Such intermediates are used in the present cleavage reaction.

As noted above, the present invention encompasses a process for the preparation of both the racemate and the R- and S-stereoisomers of glyceraldehyde-3-pentanide. Racemic glyceraldehyde-3-pentanide may be produced from either the R- or S-stereoisomer of that compound by increasing the pH of an aqueous solution of either stereoisomer above pH 10.0. Extraction of such aqueous solution, followed by distillation of the organic extract, will then provide racemic glyceraldehyde-3-pentanide.

The reaction product of the present invention can be used to synthesize drugs, agricultural chemicals and naturally occurring substances. Such synthesis is accomplished using the synthetic schemes described in United States Patent Nos. 4,526,988 and 4,567,282, with the pentanide compound of the present invention replacing the acetonide compound employed in the patents.

The following examples illustrate specific aspects of the present invention. The Examples are not intended to limit the scope of the invention in any respect and should not be so construed.

### Example 1

### 5,6-O-(3-Pentylidene)-L-gulono-1,4-lactone

To a room temperature slurry of L-gulono-1,4-lactone (25.0 g, 140.0 mmol) and camphorsulfonic acid (0.5 g) in 125 ml of dimethylformamide were added 33.3 g (253.0 mmol) of 3,3-dimethoxypentane. The resulting solution was stirred for 48 hours. Triethylamine (1 ml) was added and volatiles were removed, with heating, to provide a crude oil. Toluene (375 ml) was added to the hot oil and a precipitate began to form almost immediately. The oil/toluene mixture was cooled to 0°C and filtered. The filter cake, after being washed with cold (0°C) toluene, was dried in vacuo at 60°C for 3 hours to afford 25.93 g of title compound as a white solid. m.p. 148.5-149.5°C.
[α]_{D} = + 34.4°C (C=0.978, methanol)

| Analysis calculated for C₁₁H₁₈O₆: | | |
|---|---|---|
| Calc.: | C, 53.56; | H, 7.37; |
| Found: | C, 53.91; | H, 7.50. |

### Example 2

### S-Glyceraldehyde-3-pentanide

To a room temperature slurry of potassium periodate (19.65 g, 85.2 mmol) and potassium bicarbonate (8.54 g, 85.4 mmol) in 50 ml of water was added, dropwise at a rate which kept the reaction mixture's temperature below 27°C, a solution of 10.0 g (40.4 mmol) of the compound of Example 1 in 50 ml of tetrahydrofuran. The resulting slurry was stirred vigorously for 3 hours at room temperature, then cooled to about 0°C and filtered. Ethyl acetate (25 ml) was added to the filtrate. The resulting two-phase solution was warmed to room temperature, saturated with sodium chloride and then filtered again. The organic and aqueous layers of the two-phase solution were separated and the aqueous layer was extracted with 25 ml of ethyl acetate. The organic layer and the ethyl acetate extractant were combined and then dried over magnesium sulfate. Volatile constituents were removed under reduced pressure to provide an oil. This oil was transferred to a distillation vessel and placed under high vacuum (0.3 mm Hg) while heating. When material began to appear in the distillation head, chilled (5°C) fluid was circulated through the condenser while the receiver was immersed in an ice water bath. Distillation afforded 4.42 g (69% yield) of title compound as a clear oil. b.p. 43-44°C (0.3 mm Hg).
[α]_{D} = -79.4° (C=1.18, toluene)
¹HNMR: δ=9.57 (d,1H), 4.46 (m,1H), 3.97 (t,1H), 3.97 (dd,1H).

Glyceraldehyde-3-pentanide exists in an aqueous solution primarily as a mixture of the aldehyde form of glyceraldehyde-3-pentanide in equilibrium with a hydrate of the formula 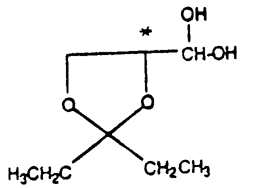 Such hydrate also has an asymetric carbon atom represented by the carbon atom labeled with an asterisk in the above formula. As such, the hydrate of glyceraldehyde-3-pentanide consists of two optically active stereoisomers; namely, the R- and S-stereoisomers, the stereochemistry of which is set forth below:

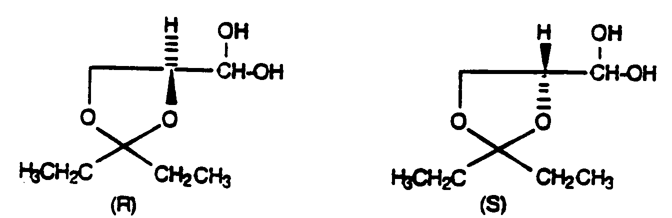

Recovery of glyceraldehyde-3-pentanide from an aqueous solution of same may be accomplished by extracting the aqueous solution with an organic solvent (after saturating the aqueous solution with sodium chloride). Such extraction provides an organic solvent solution consisting primarily of a mixture of the aldehyde form of glyceraldehyde-3-pentanide in equilibrium with the above-described hydrate. The extraction may be acomplished in good yield with stereochemistry intact. In reactions where anhydrous glyceraldehyde-3-pentanide is not required (i.e., the hydrated form of glyceraldehyde-3-pentanide may be employed), the above described organic solvent solution may be used to synthesize useful drugs, agricultural chemicals and naturally occurring substances directly. However, if anhydrous glyceraldehyde-3-pentanide is required to synthesize such useful compounds, organic solvent removal followed by distillation of the resulting residue in vacuo will provide anhydrous glyceraldehyde-3-pentanide in 70-80% yield.

## Claims

1. A process for preparing glyceraldehyde-3-pentanide which comprises reacting a compound of the formula 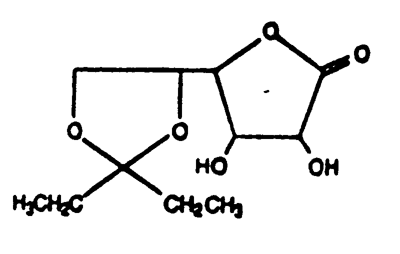 with a cleavage reagent selected from the group consisting of
a) potassium periodate and a base, and
b) dipotassium paraperiodate;
wherein the pH of the reaction solution is maintained at between about 5.0 to about 9.0.

2. A process of Claim 1 wherein the cleavage reagent is potassium periodate and a base.

3. A process of Claim 2 wherein said base is monobasic potassium phosphate, dibasic potassium phosphate, tri-basic potassium phospate, potassium carbonate, potassium bicarbonate or potassium hydroxide.

4. A process of Claim 3 wherein said base is potassium hydroxide or potassium bicarbonate.

5. A process of Claim 1 wherein R-glyceraldehyde-3-pentanide is produced.

6. A process of Claim 1 wherein S-glyceraldehyde-3-pentanide is produced.

## Patentansprüche

1. Verfahren zur Herstellung von Glyceraldehyd-3-pentanid, gekennzeichnet durch Umsetzung einer Verbindung der Formel 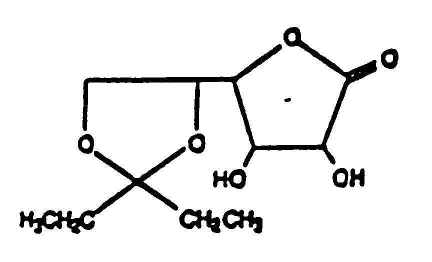 mit einem Spaltreagens, das ausgewählt ist aus der Gruppe, die besteht aus
a) Kaliumperiodat und einer Base, und
b) Dikaliumparaperiodat,
wobei der pH-Wert der Reaktionslösung auf zwischen etwa 5,0 und etwa 9,0 gehalten wird.

2. Verfahren nach Anspruch 1, worin das Spaltreagens Kaliumperiodat und eine Base ist.

3. Verfahren nach Anspruch 2, worin die Base monobasisches Kaliumphosphat, dibasisches Kaliumphosphat, tribasisches Kaliumphosphat, Kaliumcarbonat, Kaliumbicarbonat oder Kaliumhydroxid ist.

4. Verfahren nach Anspruch 3, worin die Base Kaliumhydroxid oder Kaliumbicarbonat ist.

5. Verfahren nach Anspruch 1, worin R-Glyceraldehyd-3-pentanid hergestellt wird.

6. Verfahren nach Anspruch 1, worin S-Glyceraldehyd-3-pentanid hergestellt wird.

## Revendications

1. Procédé pour préparer du 3-pentanide de glycéraldéhyde, qui comprend la réaction d'un composé répondant à la Formule 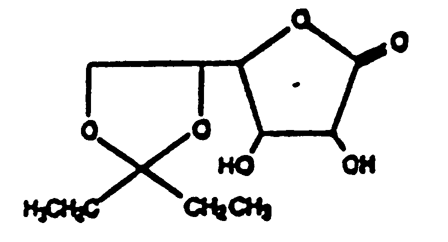 avec un réactif de clivage choisi dans le groupe constitué
a) de periodate de potassium et d'une base, et
b) de paraperiodate de dipotassium ;
dans lequel le pH de la solution réactionnelle est maintenu à une valeur entre environ 5,0 à environ 9,0.

2. Procédé selon la revendication 1, dans lequel le réactif de clivage est le periodate de potassium et une base.

3. Procédé selon la revendication 2, dans lequel ladite base est le phosphate de potassium monobasique, le phosphate de potassium dibasique, le phosphate de potassium tribasique, le carbonate de potassium, le bicarbonate de potassium ou l'hydroxyde de potassium.

4. Procédé selon la revendication 3, dans lequel ladite base est l'hydroxyde de potassium ou le bicarbonate de potassium.

5. Procédé selon la revendication 1, dans lequel le 3-pentanide de R-glycéraldéhyde est produit.

6. Procédé selon la revendication 1, dans lequel le 3-pentanide de S-glycéraldéhyde est produit.
